# EUROPEAN PATENT APPLICATION

(11) **EP 2 554 108 A1**
(43) Date of publication of application: **06.02.2013**
(21) Application number: 11759088.5
(22) Date of filing: 10.02.2011
(51) Int. Cl.: A61B 5/00, A61B 5/11

(54) **ORGANISM INFORMATION DETECTION DEVICE**

(30) Priority: 26.03.2010 JP 2010071693
(71) Applicant: Aisin Seiki Kabushiki Kaisha, Aichi 448-8650 (JP)
(72) Inventor: KOGURE, Shunsuke, Kariya-shi, Aichi 448-8650 (JP)
(74) Representative: Intès, Didier Gérard André
(86) International application number: PCT/JP2011/052837
(87) International publication number: WO 2011/118280

(57) **Abstract**

In order to provide a biological information detection apparatus capable of minimizing omission and errors in detecting a biological signal, a biological information detection apparatus is provided with: a plurality of detection units arranged on a support for supporting a living body, and adapted for outputting signals corresponding to vibration of the living body to be detected; extraction units for extracting signals in a predetermined frequency range corresponding to vibration of the living body to be detected; a timing detection unit for detecting a vibration timing of individual feature points from the signals extracted by each of the extraction units; a timing storage unit for storing a storage vibration timing that is the vibration timing detected by the timing detection unit; a determination unit for determining whether or not the detection vibration timing conforms to the stored vibration timing; and a timing output unit for outputting, as biological information about the living body, detection vibration timing in a case where the detection vibration timing conforms to the stored vibration timing.

## Description

### TECHNICAL FIELD

The present invention relates to a biological information detection apparatus for detecting biological information including biological (living body) vibrations such as respiration, heartbeat, body movement, etc.

### BACKGROUND ART

There is disclosed a technique in which an biological information detection apparatus for detecting respiration, heartbeat, body movement, or another form of vibration of a living body supported on a bed, mat, sheet, or other support comprises: pressure-sensing means for detecting fluctuation in pressure occurring in the living body; and control means for disposing the pressure-sensing means on a pressure-receiving unit for receiving the pressure of the living body, processing signals from the pressure-sensing means, and detecting signals from the living body; the pressure-sensing means being disposed with a locally varying disposition density in the pressure-receiving unit (for example, refer to Patent Document 1).

### PRIOR ART DOCUMENTS

### PATENT DOCUMENTS

Patent Document 1: Japanese Unexamined Patent Application Publication No. 2007-185409

### SUMMARY OF THE INVENTION

### OBJECT TO BE SOLVED BY INVENTION

According to the biological information detection apparatus described in Patent Document 1, in instances when, for example, the pressure-sensing means is disposed on a bed, the pressure-sensing means is disposed on a portion where the necessary signals can readily be obtained; i.e., in a position proximal to the chest when the respiration incidence timing is to be detected, so that the disposition density of the pressure-sensing means is increased. However, when the living body is positioned in a low-disposition-density location of the pressure-sensing means, the desired biological signals are less readily detected. Accordingly, problems are presented in that omission and errors occur in the detection of the timing of the biological signals.

In addition, detection errors may result from redundant detection occurring at separate positions and times, such as when biological vibration from the same vibration source is detected by pressure-sensing means in the vicinity of the vibration source, and vibration transmitted from the vibration source is detected by pressure-sensing means set apart from the vibration source.

With the foregoing problems in view, it is an object of the invention to provide a biological information detection apparatus enabling omission and errors in detecting biological signals to be minimized, and biological vibrations to be detected with higher accuracy.

### MEANS FOR ACHIEVING OBJECT

An biological information detection apparatus according to one aspect of the present invention comprises: a plurality of detection units arranged on a support for supporting a living body, and adapted for outputting signals that correspond to vibration to be detected; extraction units for extracting signals in a predetermined frequency range for the signals outputted from each of the detection units, the extracted signals corresponding to vibration of the living body to be detected; a timing detection unit for detecting, as a detection vibration timing, a vibration timing having individual feature points from the signals extracted by each of the extraction units; a timing storage unit for storing, as a stored vibration timing, the detection vibration timing detected by the timing detection unit; a determination unit for determining whether or not the detection vibration timing conforms to the stored vibration timing; and a timing output unit for outputting, as biological information about the living body, the detection vibration timing detected for at least one detection unit in a case where the detection vibration timing conforms to the stored vibration timing.

According to the aspect described above, the determination unit determines whether or not the detection vibration timing of signals where a predetermined frequency range has been extracted from the signals outputted by each of the detection units in accordance with vibration of the living body to be detected conforms to the stored vibration timing stored in the timing storage unit. As a result, in a case where the detection vibration timing conforms to the stored vibration timing, the detection vibration timing detected for at least one of the detection units is outputted as biological information about the living body by the timing output unit. Therefore, vibration of the living body to be detected can be ascertained more accurately without vibration (noise) caused by factors other than biological vibration.

The apparatus preferably further comprises a timing update unit for updating the stored vibration timing in accordance with the detection vibration timing detected by the timing detection unit.

According to the aspect described above, the stored vibration timing is updated in accordance with the detection vibration timing detected by the timing detection unit. Accordingly, even in cases such as those in which the detection vibration timing detected by the detection units does not conform to the stored vibration timing stored in the timing storage unit regardless of the presence of biological vibration, the stored vibration timing can be updated in accordance with the detection vibration timing detected by the detection units. The detection vibration timing and the updated stored vibration timing can be compared, and biological vibration can be detected with greater accuracy regardless of the stored vibration timing stored in advance.

The timing update unit preferably updates the stored vibration timing in accordance with a plurality of the detection vibration timings when the feature points of the plurality of the detection vibration timings are within a predetermined range, for each of the detection vibration timings detected by the timing detection unit.

According to the aspect described above, the stored vibration timing is updated in accordance with a plurality of detection vibration timings when the feature points of the plurality of detection vibration timings are within a predetermined range for each of the detection vibration timings detected by the timing detection unit. Accordingly, the stored vibration timing can be updated to more accurate feature-point vibration timing in conjunction with the vibration timing of feature points of actually extracted signals. Since the stored vibration timing is not updated in a case where the plurality of feature points includes feature points that fall outside of the predetermined range, no updating is performed on the stored vibration timing in a direction moving away from the vibration timing of feature points of actually extracted signals. Accordingly, actual biological vibrations can be more accurately detected.

### BRIEF DESCRIPTION OF THE DRAWINGS

FIG. 1 is a block diagram of the configuration of a biological information detection apparatus;
FIG. 2 is a view illustrating an example in which a biological information detection apparatus is used in a vehicle seat;
FIG. 3 is a flowchart of the operation of a biological information detection apparatus according to a first embodiment;
FIG. 4 is a graph of a signal outputted from each of a first low-pass filter to a fifth low-pass filter in cases where vibration of a living body performing thoracic respiration was measured;
FIG. 5 is a graph showing a signal outputted from each of a first low-pass filter to a fifth low-pass filter in a case where vibrations of a living body performing abdominal respiration were measured; and
FIG. 6 is a flowchart of the operation of a biological information detection apparatus according to a second embodiment.

### MODES OF EMBODYING THE INVENTION

Embodiments will be described below with reference to the accompanying drawings.

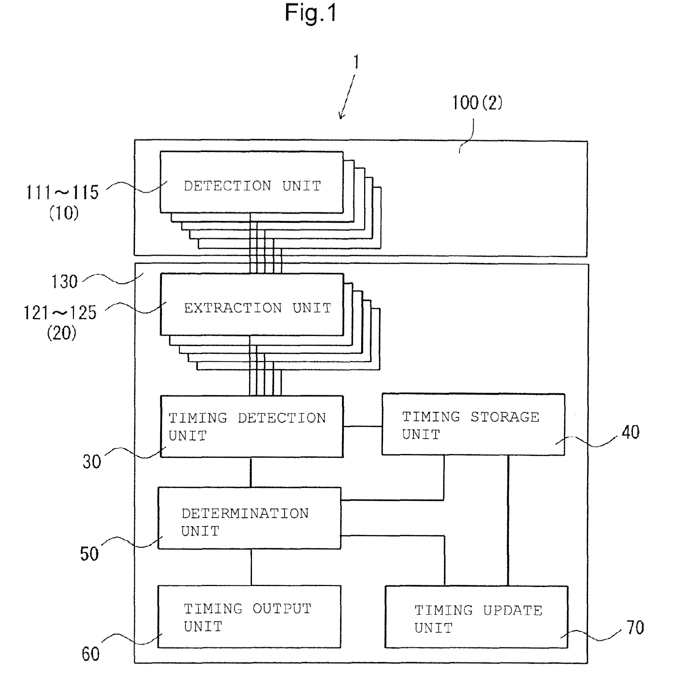

FIG. 1 is a block diagram of a configuration of the biological information detection apparatus according to the present embodiment.

A plurality of detection units 10 are arranged on a support 2 for supporting a living body, and signals corresponding to detected vibration are outputted by the detection units 10. For every signal outputted by each of the detection units 10, extraction units 20 extract signals in a predetermined frequency range corresponding to the vibration of the living body being detected. A timing detection unit 30 detects, as detection vibration timing, the vibration timing of individual feature points from the signals extracted by each of the extraction units 20. A timing storage unit 40 stores the detection vibration timing detected by the timing detection unit 30 as a stored vibration timing. A determination unit 50 determines whether or not the detection vibration timing conforms to the stored vibration timing. A timing output unit 60 outputs, as biological information about the living body, the detection vibration timing detected by at least one of the detection units 10 in a case where the detection vibration timing conforms to the stored vibration timing.

The plurality of detection units 10 are provided to the support 2 for supporting the living body. Used for the detection units 10 are a vibration sensor for outputting electrical signals in accordance with respiration, heartbeat, body movement, or other vibration produced by the living body; a pressure sensor for outputting electrical signals in accordance with pressure applied to the detection units 10 by vibration from the living body; and other sensors.

The extraction units 20 output signals obtained by extracting, from the detection signals outputted by the detection units 10, electrical signals of some or all of a frequency range (a predetermined frequency range), the frequency range having minimum and maximum values of frequencies predicted from the respiration, heartbeat, body movement, and other vibration produced by the living body. In a case where the extraction unit 20 functions as a low-pass filter, a cutoff frequency is set to be at least higher than the minimum value of the frequency predicted from the respiration, heartbeat, body movement, and other vibration produced by the living body.

Electrical signals in a frequency range, that has minimum to maximum values predicted from biological vibration, pass through the extraction unit 20 when the cutoff frequency is set higher than the maximum value of the frequency predicted from the respiration, heartbeat, body movement, and other vibration produced by the living body. In a case where the extraction unit 20 functions as a band-pass filter, the frequency range through which the signals can pass is set so as to include some or all of the frequencies, ranging from the minimum to maximum values, predicted from the respiration, heartbeat, body movement, or other vibration produced by the living body.

The timing detection unit 30 detects the detection vibration timing, which is the vibration timing of each of the feature points, from the signals extracted by each of the extraction units 20. Examples of feature points include the extreme value (peak) of the signal, the point at which the voltage of the signal switches from negative to positive, the point at which a predetermined extreme value (peak) of the signal is exceeded, and The like. The timing detection unit 30 is described herein as detecting the extreme value (peak) as a feature point. Detection of the detection vibration timing is performed for each of the detection units 10.

The timing storage unit 40 stores, as stored vibration timing, the vibration timing at which a feature point is detected by each of the detection units 10. The vibration timing at which vibration produced by the living body to be detected is detected by the detection units 10 is determined using the positional relationship (distance) between the vibration source of the biological vibration and the individual detection unit 10. The source of the biological vibration differs according to whether the vibration is caused by respiration or from the heartbeat, or whether the respiration is caused by abdominal respiration or thoracic respiration; therefore, the vibration timing detected by each of the detection units 10 differs according to the type of vibration of the living body to be detected. Accordingly, the timing storage unit 40 stores stored vibration timing for each of the detection units 10, and the stored vibration timing corresponds to the vibration of the living body to be detected.

In a case where there are a plurality of types of vibration of the living body to be detected, stored vibration timing for individual detection categories (e.g., abdominal respiration and thoracic respiration) are stored. Vibration (noise) created by a factor other than biological vibration tends to simultaneously cause the entire support to vibrate. Specifically, vibration in which a feature point of noise-derived vibration is detected by each of the detection units 10 occurs at substantially the same time, and is different from the stored vibration timing that correspond to biological vibration for each of the detection units 10.

The determination unit 50 compares the detection vibration timing detected for each of the detection units 10 by the timing detection unit 30 and the stored vibration timing stored for each of the detection units 10 by the timing storage unit 40, and determines whether or not the detection vibration timing and the stored vibration timing conform to each other. Whether or not the timings conform to each other is determined by whether or not the discrepancy of the detection vibration timing detected for each of the detection units 10 by the timing detection unit 30, relative to the stored vibration timing stored for each of the detection units 10 by the timing storage unit 40, falls within a predetermined range.

For the stored vibration timing, it is possible to set a predetermined range (permissible range) for when a determination is made as to whether or not there is conformance with a particular timing, or set a certain range (permissible range) for the stored vibration timing itself, In a case where there are a plurality of types of vibration produced by living body to be detected, the stored vibration timing is compared for each living body to be detected.

For comparison, the determination unit 50 calculates the difference between the detection vibration timing of each of the detection units 10 and a reference detection vibration timing, the reference detection vibration timing being a vibration timing detected by the timing detection unit 30 from signals outputted by at least one detection unit 10 and extracted by the extraction unit 20. In addition, the determination unit 50 calculates the difference between the stored vibration timings of each of the detection units 10 and a reference stored vibration timing, the reference stored vibration timing being a stored vibration timing for at least one detection unit 10. The difference between the detection vibration timing of each of the detection units 10 and the reference detection vibration timing may be calculated by the timing detection unit 30. In addition, the difference between the stored vibration timing for each of the detection units 10 and the reference stored vibration timing may be stored in the timing storage unit 40.

The timing output unit 60 outputs, as biological information about the living body, the detection vibration timing for at least one detection unit 10 in a case where the determination unit 50 determines that the detection vibration timing and the stored vibration timing conform to each other. At this time, the timing output unit 60 may output the average of the detection vibration timings for the plurality of detection units 10.

The timing output unit 60 thus outputs the detection vibration timing in a case where a determination has been made that the detection vibration timing detected by the timing detection unit 30 and the stored vibration timing stored in the timing storage unit 40 correspond to each other. Biological vibration to be detected can accordingly be more accurately obtained without vibration (noise) caused by factors other than biological vibration. Accordingly, the biological information detection apparatus 1 can detect biological information (respiration rate per unit time, whether respiration is occurring in the abdomen or thorax, heart rate per unit time, variations thereof over time, and other data) on the basis of more accurately obtained biological vibration information.

The timing storage unit 40 may store in advance the stored vibration timings for each of the detection units 10 that correspond to the biological vibrations to be detected, and may be configured so as to store the detection vibration timings detected by the timing detection unit 30 and compare these timings with those obtained during a later timing detection. In addition, in a case where the detection vibration timing detected by the timing detection unit 30 is stored, detection of the detection vibration timing may be performed a plurality of times, and the detection vibration timing may be determined and stored on the basis of the results thereof.

The biological information detection apparatus 1 may furthermore be provided with a timing update unit 70 for updating the stored vibration timing in accordance with the detection vibration timing detected by the timing detection unit 30. An accordance with the plurality of detection vibration timings, the timing update unit 70 updates the stored vibration timing for the individual detection vibration timing detected by the timing detection unit when the feature points of the plurality of detection vibration timings are within a predetermined range.

When the stored vibration timing is updated by the timing update unit 70, the stored vibration timing is updated in accordance with the detection vibration timing for each of the detection units 10, and the detection vibration timing for each of the detection units 10 is compared with the updated stored vibration timing even in cases such as those in which the detection vibration timing does not correspond with the stored vibration timing stored in advance in the timing storage unit 40, despite the fact that the vibrations are created by biological vibrations. Accordingly, biological vibrations can be detected with greater accuracy without dependence on the stored vibration timings stored in advance.

In a case where the detection vibration timing detected in accordance with the vibration of the living body is detected in a smaller range than the range in which it is determined that the detection vibration timing conform to the stored vibration timing, updating can be performed by the timing update unit 70 so that the range in which the detection vibration timings are determined as conforming to the stored vibration timings is smaller. Detection can therefore be performed more accurately in accordance with the actual biological vibration.

In addition, for each of the signals extracted by the extraction unit 20, the stored vibration timing is updated in accordance with the vibration timing of a plurality of feature points when the vibration timing of the plurality of feature points is within a predetermined range; therefore, the stored vibration timing can be updated to more accurate feature-point vibration timing in conjunction with the vibration timing of the feature points of the actually extracted signals. The stored vibration timing is not updated in a case where the plurality of feature points includes feature points that fall outside of the predetermined range, and accordingly no update is performed on the stored vibration timing in a direction moving away from the vibration timing of the feature points of actually extracted signals. Accordingly, actual biological vibration can be more accurately detected.

There follows a description of an example in which the biological information detection apparatus of the present embodiment is used in a vehicle seat.

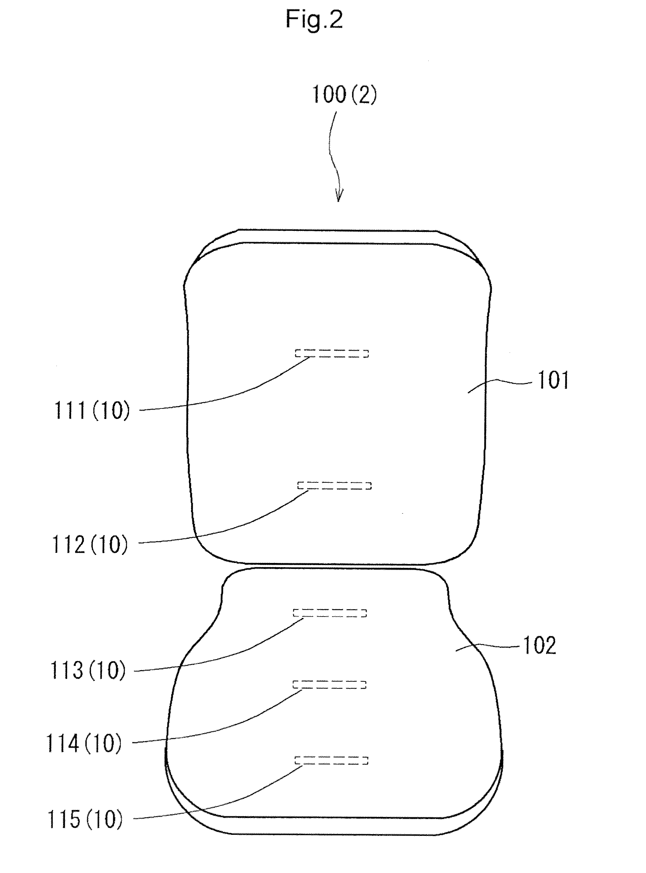

FIG. 2 is a view illustrating an example in which the biological information detection apparatus according to the present embodiment is used in a vehicle seat 100.

A first pressure sensor 111, a second pressure sensor 112, a third pressure sensor 113, a fourth pressure sensor 114, and a fifth pressure sensor 115 are arranged on a vehicle seat 100 as detection units 10 for detecting respiration, heartbeat, body movement, and other vibration produced by a living body sitting on a vehicle seat 100, which is used as the support 2, as shown in FIG. 2. The first pressure sensor 111 and the second pressure sensor 112 are arranged on a backrest 101; and the third pressure sensor 113, the fourth pressure sensor 114, and the fifth pressure sensor 115 are arranged on a seat part 102.

Each of the first pressure sensor 111 to the fifth pressure sensor 115 is connected to channels of an electronic circuit 130, as shown in FIG. 1. The electronic circuit 130 is provided with a program for functioning as the extraction unit 20, the timing detection unit 30, the timing storage unit 40, the determination unit 50, and the timing output unit 60. As described herein, the extraction unit 20 is made to function as a low-pass filter, and is provided with a first low-pass filter 121 to a fifth low-pass filter 125 for extracting signals filtered from signals outputted from the first pressure sensor 111 to the fifth pressure sensor 115. The cutoff frequency of the first low-pass filter 121 to the fifth low-pass filter 125 is set in accordance with the frequency of vibration created by respiration of the living body.

The extraction unit 20 may be configured using an analog filter (low-pass filter or band-pass filter), without dependence on the electronic circuit 130.

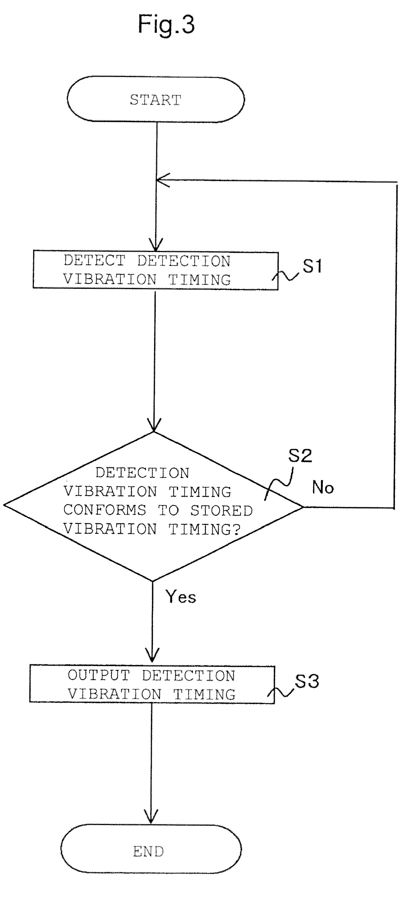

FIG. 3 is a flowchart of the operation of the biological information detection apparatus according to a first embodiment. The flowchart of FIG. 3 represents a routine for performing a process in which the detection vibration timing is detected from the signals extracted by the first low-pass filter 121 to the fifth low-pass filter 125, and the detection vibration timing is outputted in a case where a determination has been made that the detected detection vibration timing corresponds to the stored vibration timing. In step S1, the electronic circuit 130 detects the detection vibration timing from the signals extracted by the first low-pass filter 121 to the fifth low-pass filter 125.

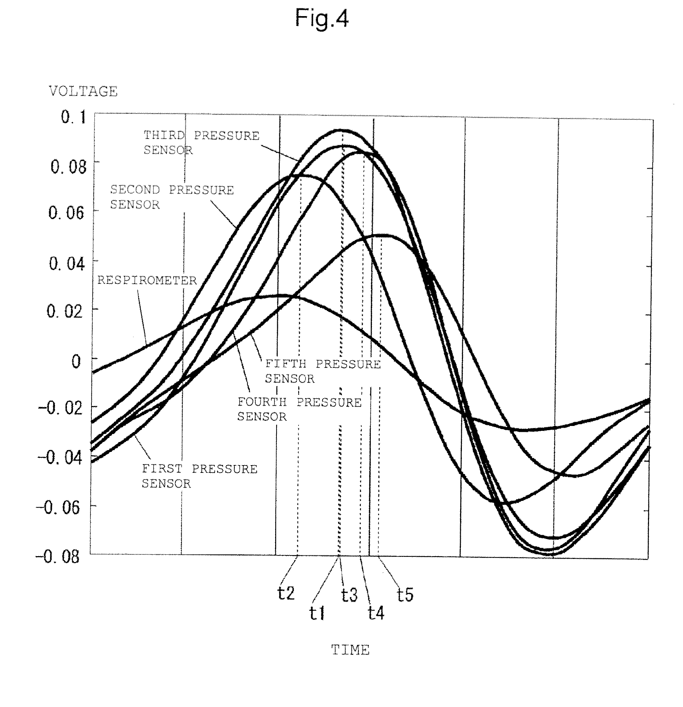

FIG. 4 is a graph of the signals of the first pressure sensor 111 to the fifth pressure sensor 115 extracted from the first low-pass filter 121 to the fifth low-pass filter 125 in a case where vibration of living body performing thoracic respiration was measured. The horizontal axis is time, and the vertical axis is the voltage of the extracted signals. In addition, the results obtained by performing measurements using a respirometer are shown together with measurements of the biological vibration on the same graph. The electronic circuit 130 detects the detection vibration timing, which is the vibration timing of the feature points, from each of the signals. The feature points detected by the electronic circuit 130 are the extreme values (peaks) of the signals, and the detection vibration timings t1 to t5 are detected for the first pressure sensor 111 to the fifth pressure sensor 115, respectively.

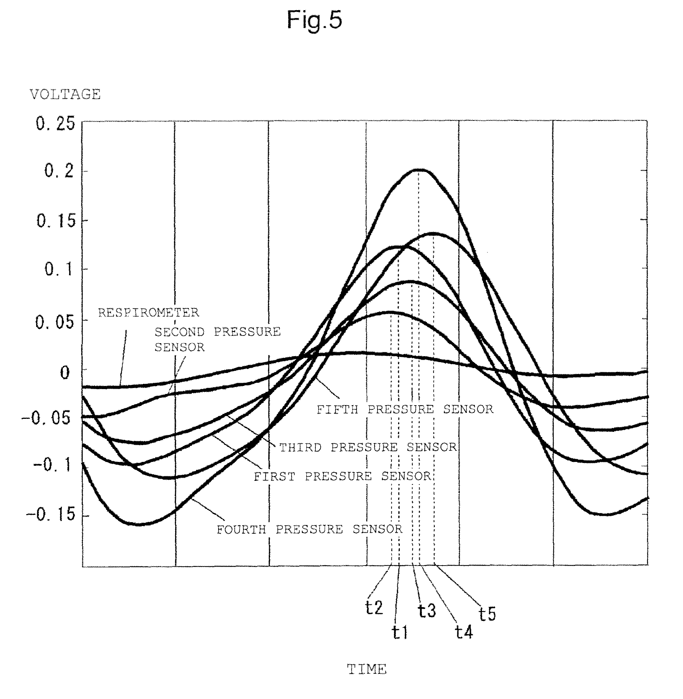

FIG. 5 is a graph showing signals of the first pressure sensor 111 to the fifth pressure sensor 115 extracted from the first low-pass filter 121 to the fifth low-pass filter 125 in a case where vibration of a living body performing abdominal respiration was measured. The horizontal axis is the time, and the vertical axis is the voltage of the extracted signals. The results obtained by performing measurements using a respirometer are shown together with measurements of the biological vibrations on the same graph. The electronic circuit 130 detects the detection vibration timing, which is the vibration timing of the feature points, from each of the signals. Here, the feature points detected by the electronic circuit 130 are the extreme values (peaks) of the signals, and the detection vibration timings t1 to t5 are detected for the first pressure sensor 111 to the fifth pressure sensor 115, respectively.

It follows from FIGS. 4 and 5 that the detection vibration timings t1 to t5 of each of the first pressure sensor 111 to the fifth pressure sensor 115 differ according to the type of respiration performed by the living body. The electronic circuit 130 stores the stored vibration timing that is set on the basis of data obtained from measuring in advance, for each of the first pressure sensor 111 to the fifth pressure sensor 115, vibration timing for which signals extracted when biological vibration was obtained were used as feature points, in accordance with the type of vibration (or a plurality of types in the case of a plurality of types of vibration) of the living body detected.

The stored vibration timing for the first pressure sensor 111 may be established as a reference stored vibration timing (t1=0), and the stored vibration timing for the second pressure sensor 112 to the fifth pressure sensor 115 may be stored in the electronic circuit 130 as the difference between the reference stored vibration timing and the stored vibration timing of the second pressures sensor 112 to the fifth pressure sensor 115.

In step S2, the electronic circuit 130 compares the detection vibration timings t1 to t5 detected in step S1 to the stored vibration timing. In order to perform this comparison, the electronic circuit 130 establishes the detection vibration timing for the first pressure sensor 111 as the reference detection vibration timing, calculates the difference between the reference detection vibration timing and the detection vibration timing t1 for the second pressure sensor 112 to the fifth pressure sensor 115, and compares the reference stored vibration timing for the first pressure sensor 111 (t=0) and the stored vibration timing (difference between the reference stored vibration timing and the stored vibration timing) for the second pressure sensor 112 to the fifth pressure sensor 115. The electronic circuit 130 determines whether or not the detection vibration timing for each of the pressure sensors 111 to 115 conforms to the stored vibration timing (whether or not the discrepancy between the detection vibration timing and the stored vibration timing is within a predetermined range).

Once the electronic circuit 130 determines whether the detection vibration timing for each of the pressure sensors 111 to 115 conforms to the stored vibration timing, at least one detection vibration timing of the first pressure sensor 111 to the fifth pressure sensor 115 (a detection vibration timing that is the vibration timing in which a feature point is detected, and not the difference between the detection vibration timing and the reference detection vibration timing) is outputted in step S3, and the sequential process in the routine is ended. Here, the outputted detection vibration timing may be at least one detection vibration timing of the first pressure sensor 111 to the fifth pressure sensor 115, and may be the average of the detection vibration timing of each of the first pressure sensor 111 to the fifth pressure sensor 115. The detection vibration timing outputted from the electronic circuit 130 can be used, for example, for measuring the heart rate and respiration rate per unit time, and the like, or for other purposes, in a later process.

When a determination has been made that the detection vibration timing for each of the pressure sensors 111 to 115 does not conform to the stored vibration timing, the electronic circuit 130 then detects the detection vibration timing.

Thus, in a case where the detection vibration timing detected by the first pressure sensor 111 to the fifth pressure sensor 115 and the stored vibration timing conform to each other, the electronic circuit 130 outputs the detection vibration timing. Therefore, vibration of the living body to be detected can be more accurately ascertained without vibration (noise) caused by factor other than biological vibration.

In a case where the biological information detection apparatus is applied to a vehicle seat, as in the first embodiment, there is a tendency, for example, for vibration created by the vehicle going over a bump or the like to vibrate the entire vehicle seat at the same time. The vibration detected by the first pressure sensor 111 to the fifth pressure sensor 115 therefore occurs at the same time (the difference between the detection vibration timing for each of the second pressure sensor 112 to the fifth pressure sensor 115 and the reference detection vibration timing is zero in every case), and the detection vibration timing is different from the stored vibration timing. The detection vibration timing is accordingly not outputted by the electronic circuit 130.

Thus, since noise can be removed, the biological information detection apparatus 1 of the first embodiment can detect biological information (respiration rate per unit time, whether respiration is occurring in the abdomen or thorax, heart rate per unit time, variations thereof over time, and the like) on the basis of vibration information obtained from biological vibration.

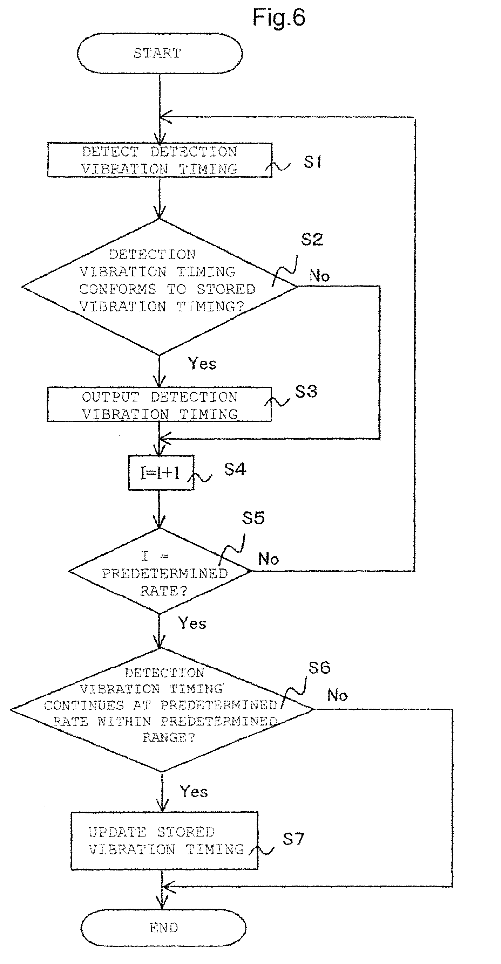

FIG. 6 is a flowchart of the operation of the biological information detection apparatus 1 according to a second embodiment. Since the second embodiment is substantially the same as the first embodiment, the description will focus on the differences between the first and second embodiments. The flowchart of FIG. 6 indicates a routine for performing a process in which the stored vibration timing is updated in accordance with the detection vibration timing.

In the biological information detection apparatus 1 according to the second embodiment, the electronic circuit 130 detects the detection vibration timing in step S1, and determines whether the detection vibration timing conforms to the stored vibration timing in step S2. As a result, in a case where the timings are determined to conform to each other, the detection vibration timing is output in step S3, and the process is advanced to step S4. The process moves immediately to step S4 in a case where it is determined that the timings do not conform to each other.

In step S4, the electronic circuit 130 counts a detection rate I of the detection vibration timing, and in step S5 determines whether or not the detection rate I is a predetermined rate. The electronic circuit 130 advances the process to step S6 in a case where it was determined that the detection rate I is the predetermined rate, and returns the process to step S1 in a case where it was determined that the detection rate I is not the predetermined rate. In step S6, a determination is made as to whether or not the detection vibration timing continues at the predetermined rate within a predetermined range.

In a case where it was determined that the detection vibration timing continued at the predetermined rate within the predetermined range, the electronic circuit 130 advances the process to step S7, updates the stored vibration timing in accordance with the detection results for the predetermined rate of the detection vibration timing, and ends the successive process in the routine. In a case where a determination is made that the detection vibration timing does not continue at the predetermined rate within the predetermined range, the electronic circuit 130 ends the successive process in the routine.

Updating the stored vibration timing is performed by, for example, calculating the average of the detection vibration timings for the predetermined rate, and storing the average as a new stored vibration timing. In step S2, the predetermined range (permissible range) used when the electronic circuit 130 determines whether or not the detection vibration timing corresponds to the stored vibration timing can be updated to a range used for the electronic circuit 130 to determine in step S6 whether the detection vibration timing continues at the predetermined rate within the predetermined range.

Even in cases such as those in which, when the stored vibration timing is updated, the detection vibration timing for each of the first pressure sensor 111 to the fifth pressure sensor 115 does not conform to the stored vibration timing stored in advance regardless of the presence of biological vibration, the stored vibration timing is updated in response to the detection vibration timing for each of the first pressure sensor 111 to the fifth pressure sensor 115, and the detection vibration timing and the updated stored vibration timing is compared. Accordingly, biological vibration can be detected with greater accuracy regardless of the stored vibration timing stored in advance.

When vibration timing of a plurality of feature points is within a predetermined range for signals extracted by the extraction unit 20, the stored vibration timing is updated in accordance with the vibration timing of the plurality of feature points; therefore, the stored vibration timing can be updated to more accurate feature-point vibration timing in conjunction with the vibration timing of feature points of actually extracted signals, The stored vibration timing is not updated in a case where the plurality of feature points includes feature points that fall outside of the predetermined range; therefore, no update is performed on the stored vibration timing in a direction moving away from the vibration timing of feature points of actually extracted signals. Accordingly, actual biological vibration can be more accurately detected.

In a case where the stored vibration timing for each of the first pressure sensor 111 to the fifth pressure sensor 115 is stored in advance in the electronic circuit 130, the predetermined range (permissible range) when it is determined that there is conformance with the stored vibration timing is typically set to be of a certain large magnitude in accordance with the various physiques and states of the living body. However, updating the stored vibration timing and the permissible range relative to the stored vibration timing (or the range of the stored vibration timing) in accordance with the detection vibration timing causes the magnitude of the detection vibration timing determined to conform to the stored vibration timing to be reduced in accordance with the actual physique and state of the living body. Therefore, biological vibration can be more accurately detected.

### DESCRIPTION OF REFERENCE MARKS/MUMERALS

- 1: Biological information detection apparatus
- 2: Support
- 10: Detection unit
- 20: Extraction unit
- 30: Timing detection unit
- 40: Timing storage unit
- 50: Determination unit
- 60: Timing output unit
- 70: Timing update unit

## Claims

1. A biological information detection apparatus comprising:
a plurality of detection units arranged on a support for supporting a living body, and adapted for outputting signals that correspond to vibration to be detected;
extraction units for extracting signals in a predetermined frequency range for the signals outputted from each of the detection units, the extracted signals corresponding to vibration of the living body to be detected;
a timing detection unit for detecting, as a detection vibration timing, a vibration timing of individual feature points from the signals extracted by each of the extraction units;
a timing storage unit for storing, as a stored vibration timing, the detection vibration timing detected by the timing detection unit;
a determination unit for determining whether or not the detection vibration timing conforms to the stored vibration timing; and
a timing output unit for outputting, as biological information about the living body, the detection vibration timing detected for at least one detection unit in a case where the detection vibration timing conforms to the stored vibration timing.

2. The biological information detection apparatus of claim 1 further comprising a timing update unit for updating the stored vibration timing in accordance with the detection vibration timing detected by the timing detection unit.

3. The biological information detection apparatus of claim 2, the timing update unit updating the stored vibration timing in accordance with a plurality of the detection vibration timings when the feature points of the plurality of the detection vibration timings are within a predetermined range, for each of the detection vibration timings detected by the timing detection unit.
